(19) Europäisches Patentamt
European Patent Office
Office européen des brevets

(11) Veröffentlichungsnummer: **0 229 381**
**B1**

(12) # EUROPÄISCHE PATENTSCHRIFT

(45) Veröffentlichungstag der Patentschrift: **30.05.90**

(51) Int. Cl.$^5$: **C 07 D 211/90,**
C 07 D 413/04,
C 07 D 401/04, A 61 K 31/44

(21) Anmeldenummer: **86117966.1**

(22) Anmeldetag: **23.12.86**

(54) **Methioninsubstituierte 1,4-Dihydropyridine, Verfahren zur Herstellung und ihre Verwendung.**

(30) Priorität: **11.01.86 DE 3600594**

(43) Veröffentlichungstag der Anmeldung:
**22.07.87 Patentblatt 87/30**

(45) Bekanntmachung des Hinweises auf die Patenterteilung:
**30.05.90 Patentblatt 90/22**

(84) Benannte Vertragsstaaten:
**AT BE CH DE ES FR GB GR IT LI NL SE**

(56) Entgegenhaltungen:
**EP-A-0 095 451**
**WO-A-84/02132**

(73) Patentinhaber: **BAYER AG**
**D-5090 Leverkusen 1 Bayerwerk (DE)**

(72) Erfinder: **Kinast, Günther, Dr.**
**Am Eckbusch 15a**
**D-5600 Wuppertal 1 (DE)**
Erfinder: **Schwenner, Eckhard, Dr.**
**Claudiusweg 3**
**D-5600 Wuppertal 1 (DE)**
Erfinder: **Kazda, Stanislav, Prof. Dr.**
**Gellertweg 18**
**D-5600 Wuppertal 1 (DE)**
Erfinder: **Knorr, Andreas, Dr.**
**Trillser Graben 10**
**D-4006 Erkrath 2 (DE)**
Erfinder: **Kayser, Michael, Dr.**
**Fleyerstrasse 231**
**D-5800 Hagen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft methioninsubstituierte 1,4-Dihydropyridine, ein Verfahren zur Herstellung und ihre Verwendung in Arzneimitteln, insbesondere in kreislaufbeeinflussenden Arzneimitteln.

Es ist bekannt, daß man 1,4-Dihydro-2,6-dimethyl-4-phenylpyridin-3,5-dicarbonsäure-diethylester erhält, wenn man 2-Benzylidenacetessigsäureethylester mit β-Aminocrotonsäureethylester oder Acetessigsäureethylester mit Ammoniak umsetzt [E. Knoevenagel, Ber. dtsch. Chem. Ges. *31*, 743 (1898)]. Außerdem ist bekannt, daß bestimmte 1,4-Dihydropyridine interessante pharmakologische Eigenschaften aufweisen (F. Bossert, W. Vater, Naturwissenschaften *58*, 578 (1971)).

Aus der Anmeldung WO-A1-84 102 132 sind bereits Dihydropyridinderivate mit pharmazeutischer Wirkung bekannt. Die erfindungsgemäßen Verbindungen unterscheiden sich jedoch in ihrer chemischen Struktur und in ihrer Wirkungsspezifität eindeutig von den Dihydropyridinen aus dem Stand der Technik.

Die vorliegende Erfindung betrifft methioninsubstituierte 1,4-Dihydropyridine der allgemeinen Formel (I)

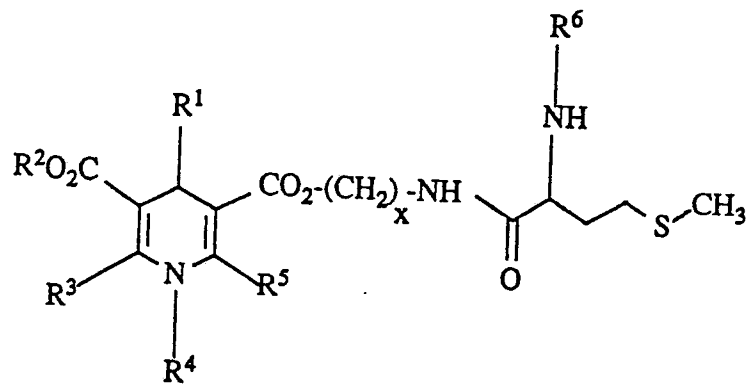

(I)

in welcher

$R^1$ für Phenyl, Thienyl, Furyl, Pyridyl, Chinolyl oder Benzoxadiazolyl steht, wobei die genannten Ringsysteme gegebenenfalls durch 1 bis 2 gleiche oder verschiedene Substituenten aus der Gruppe Phenyl, Alkyl mit 1 bis 2 Kohlenstoffatomen, Alkoxy mit 1 bis 2 Kohlenstoffatomen, Dioxymethylen, Fluor, Chlor, Brom oder Jod, Trifluormethyl, Trifluormethoxy, Difluormethoxy, Nitro, Cyano oder Azido, Trifluormethylsulfonyl oder Alkylmercapto mit 1 bis 4 Kohlenstoffatomen im Alkylrest substituiert sind,

$R^2$ einen geradkettigen, verzweigten oder cyclischen, gesättigten oder ungesättigten Kohlenwasserstoffrest mit bis zu 14 Kohlenstoffatomen darstellt, der gegebenenfalls durch ein Sauerstoffatom in der Kette unterbrochen ist und/oder der gegebenenfalls substituiert ist durch Fluor, Chlor, Brom, Jod, Cyano, Hydroxy, Acetoxy, Nitrooxy oder durch eine gegebenenfalls durch Fluor oder Chlor, Alkoxy mit 1 bis 4 Kohlenstoffatomen, Alkyl mit 1 bis 4 Kohlenstoffatomen, oder Trifluormethyl substituierte Phenyl- oder Phenoxygruppe oder durch eine α-, β- oder γ-Pyridylgruppe oder durch eine Aminogruppe, wobei diese Aminogruppe zwei gleiche oder verschiedene Substituenten aus der Gruppe Alkyl mit bis zu 4 Kohlenstoffatomen, Phenyl oder Benzyl trägt,

$R^3$ und $R^5$ gleich oder verschieden sind und jeweils für Wasserstoff, einen geradkettigen, verzweigten oder cyclischen Alkylrest mit bus zu 6 Kohlenstoffatomen, einen Phenyl- oder Benzylrest stehen, oder einen der Substituenten $R^3$ oder $R^5$ einen Alkylrest mit bis zu 4 Kohlenstoffatomen darstellt, der durch Acetoxy, Methoxy, oder Dimethoxy, Hydroxy, Amino, Phthalimido, Aminoalkoxy oder Phthalimidoalkoxy mit jeweils bis zu 4 Kohlenstoffatomen je Alkoxygruppe substituiert ist oder für die Formyl- oder Nitrilgruppe steht,

$R^4$ Wasserstoff oder einen Alkylrest mit bis zu 4 Kohlenstoffatomen bedeutet, der gegebenenfalls durch ein Sauerstoffatom in der Kette unterbrochen ist und/oder der gegebenenfalls durch einen Morpholino-Rest substituiert ist, oder einen Phenyl- oder Benzylrest darstellt,

$R^3$ für Wasserstoff oder eine Aminoschutzgruppe aus der Gruppe Vinyl, Allyl, tert.-Butyloxycarbonyl, Benzyl, Benzyloxycarbonyl, 2-Nitro- bzw. 4-Nitrobenzyl, 2-Nitro- bzw. 4-Nitro-benzyloxycarbonyl, 4-Methoxyphenyl, Formyl, Benzoyl, Acetyl, Chloracetyl, Trichloracetyl, Trifluoracetyl, Methyloxycarbonyl, Allyloxycarbonyl, Trimethyl-, Triethyl-bzw. Triphenylsilyl, tert.-Butyl-dimethylsilyl, 2,4-Dimethoxybenzyl, 2,4-Dimethoxybenzyloxycarbonyl, 4-Methoxybenzyl, 4-Methoxymethyloxyphenyl, Bis(4-Methoxyphenyl)methyl, tert.-Butoxycarbonylmethyl, Allyloxycarbonylmethyl, Methoxymethyl, Methylthiomethyl, Methoxyethoxymethyl, [2-(Trimethylsilyl)ethoxy]-methyl, 2-(Methylthiomethoxy)ethoxycarbonyl oder Tetrahydropyranyl steht, und

X für eine Zahl von 2 bis 12 steht,

und deren physiologisch unbedenklichen Salze.

Bevorzugt sind solche Verbindungen der allgemeinen Formel (I),

in welcher

$R^1$ für Phenyl, Pyridyl oder Benzoxadiazolyl steht, wobei der Phenylring durch 1 oder 2 gleiche oder verschiedene Substituenten aus der Gruppe Chlor, Trifluormethyl, Nitro oder Cyano substituiert ist,

$R^2$ einen geradkettigen, verzweigten oder cyclischen Kohlenwasserstoffrest mit bis zu 7 Kohlenstoffatomen darstellt, der gegebenenfalls durch ein Sauerstoffatom in der Kette unterbrochen ist und/oder der gegebenenfalls substituiert ist durch Fluor, Cyano, Acetoxy, Phenyl, Phenoxy, α-, β- oder γ-Pyridyl oder durch eine Aminogruppe, wobei diese Aminogruppe zwei gleiche oder verschiedene Substituenten aus der Gruppe Alkyl mit bis zu 4 Kohlenstoffatomen und Benzyl trägt,

$R^3$ und $R^5$ gleich oder verschieden sind und jeweils für einen geradkettigen oder cyclischen Alkylrest mit bis zu 6 Kohlenstoffatomen stehen, oder

einer der Substituenten $R^3$ oder $R^5$ einen Alkylrest mit bis zu 2 Kohlenstoffatomen darstellt, der durch Acetoxy-Hydroxy, Phthalimido, Amino, Phthalimidoethoxy oder Aminoethoxy substituiert ist, oder für die Formyl- oder Nitrilgruppe steht,

$R^4$ vorzugsweise für Wasserstoff oder den Morpholinoethylrest steht, und

X für eine Zahl von 2 bis 8 steht,

und deren physiologisch unbedenklichen Salze.

Physiologisch unbedenkliche Salze sind Salze der erfindungsgemäßen Verbindungen mit anorganischen und organischen Säuren. Hierzu gehören bevorzugt anorganische Mineralsäuren wie beispielsweise Salzsäure, Bromwasserstoffsäure, Phosphorsäure, Schwefelsäure oder organische Carbonsäuren, wie beispielsweise Mischsäure, Essigsäure, Maleinsäure, Fumarsäure, Zitronensäure, Apfelsäure, Bernsteinsäure oder Benzoesäure.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Raceformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Die erfindungsgemäßen Verbindungen der allgemeinen Formel (I) werden hergestellt, indem man Methioninverbindungen der allgemeinen Formel (II),

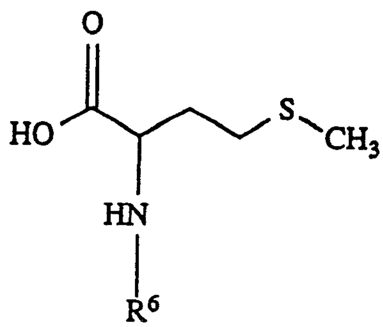

(II)

in der

$R^6$ für eine Aminoschutzgruppe mit der oben angegebenen Bedeutung steht,

nach Aktivierung der Carboxylgruppe mit Dihydropyridinen der allgemeinen Formel (III)

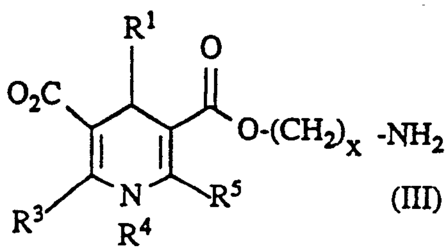

(III)

in welcher

$R^1$ bis $R^5$ und X die oben angegebene Bedeutung haben,

in einem inerten Lösungsmittel bei Temperaturen zwischen −30°C und +60°C umsetzt, gegebenenfalls vorhandene Schutzgruppen abspaltet und anschließend gegebenenfalls durch Umsetzen mit Säuren

physiologisch unbedenkliche Salze herstellt.

Die Reaktion kann durch folgende Reaktionsgleichung verdeutlich werden:

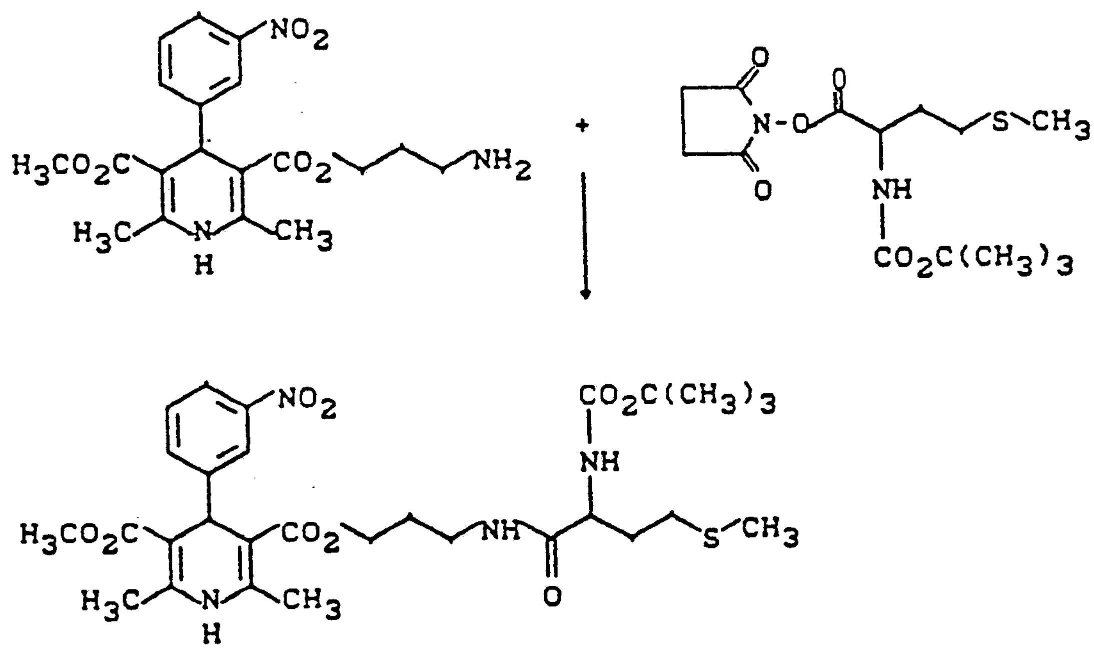

Für die Kupplung der Methioninderivate der Formel (II) an Dihydropyridine der Formel (III), kann eine große Zahl von aus der Peptidchemie bekannten Methoden verwendet werden [Houben-Weyl's "Methoden der Organischen Chemie", XV/1 Seite 40].

Es hat sich als vorteilhaft erwiesen, die Methioninderivate zu aktivieren und dann mit den Dihydropyridinen zu kuppeln. Besonders bevorzugt ist die Aktivierung als N-Succinimidylester.

Als Lösungsmittel eignen sich die üblichen inerten organischen Lösungsmittel, die sich unter den Reaktionsbedingungen nicht verändern. Hierzu gehören bevorzugt Alkohole wie Methanol, Ethanol, Propanol oder Isopropanol oder Ether wie Diethylether, Dioxan, Tetrahydrofuran oder Dimethylformamid, Hexamethylphosphorsäuretriamid, Essigester, Acetonitril oder Aceton. Es ist jedoch auch möglich, Gemische der genannten Lösungsmittel einzusetzen.

Die Reaktion wird in einem Temperaturbereich von −30°C bis +60°C, bevorzugt von −20°C bis +20°C durchgeführt.

Die Umsetzung kann bei normalem aber auch bei erhöhtem oder erniedrigtem Druck erfolgen. Im allgemeinen arbeitet man bei Normaldruck.

Es hat sich bei der Durchführung als günstig erwiesen, in einem pH-Bereich von pH 7—pH 11 zu arbeiten. Gegebenenfalls erreicht man dies durch Zugabe einer Base wie Triethylamin oder durch Zugabe von Puffersystemen wie Borat- oder Phosphatpuffer. Es ist jedoch durchaus möglich die Reaktion ohne Base bzw. Puffer durchzuführen.

Bei der Durchführung des Verfahrens werden im allgemeinen 0,1 bis 10, bevorzugt 0,5—5, besonders bevorzugt 1—1,5 Mol Dihydropyridin, bezogen auf 1 Mol Methioninderivat eingesetzt.

Die als Ausgangsstoffe verwendeten Dihydropyridine der Formel (III) sind bekannt oder können nach bekannten Methoden hergestellt werden [vgl. US—PS 3 985 758, EP—AS 151 006].

Die als Ausgangsstoffe verwendeten Methioninderivate der Formel (II) sind bekannt oder können nach bekannten Methoden hergestellt werden [Houben-Weyl's "Methoden der Organischen Chemie" XV/1 Seite 46].

Die Methioninderivate können hierbei in ihrer D-Form, L-Form oder als DL-Gemisch eingesetzt werden.

Die erfindungsgemäßen Verbindungen haben ein breites und vielseitiges pharmakologisches Wirkspektrum.

Im einzelnen konnten im Tierexperiment folgende Hauptwirkungen nachgewiesen werden:

1. Die Verbindungen bewirken bei parenteraler, oraler oder perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Die gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Verbindungen haben stark muskulär-spasmolytische Wirkungen, die an der glatten Muskulatur des Magens, Darmtrakts, des Urogenitaltraktes und des Respirationssystems deutlich werden.

6. Die Verbindungen haben eine überraschend hohe Affinität zu den 1,4-Dihydropyridin-Rezeptoren im Körper, z.B. in den Gefäßen, im Zentralen Nervensystem, im Coronarbereich und in Skelettmuskeln. Ihre Dissoziationskonstanten bei Körpertemperatur für diese Rezeptoren liegen mehr als eine 10er-Potenz niedriger als die Dissoziationskonstanten von bekannten Dihydropyridinen wie. z.B. von Nimodipin (2,6-Dimethyl-4-(3'-nitrophenyl)-1,4-dihydropyridin-3-β-methoxyethylester-5-isopropylester).

Die erfindungsgemäßen Verbindungen eignen sich aufgrund dieser Eigenschaften besonders zur Prophylaxe und Therapie der akuten und chronischen ischämischen Herzkrankheiten im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielspeise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittels als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle, (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tabletieren mitverwendet werden. Im Falle wäßriger Suspension und/oder Elixieren, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auf aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

1,4-Dihydro-2,6-dimethyl-4-(2-trifluormethylphenyl)-pyridin-3,5-dicarbonsäure-3-[2-(N-tert.-butyloxycarbonyl-L-methionyl)-aminoethyl]-ester-5-ethylester

Beispiel 1

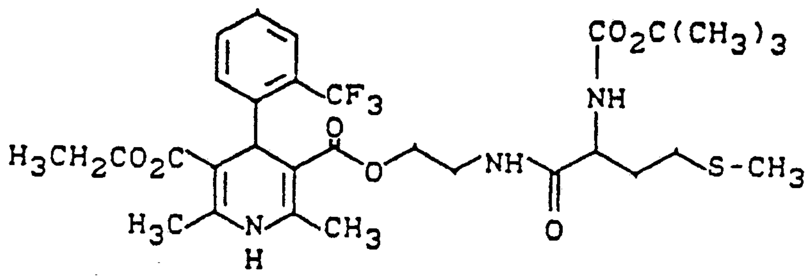

7,3 mmol tert.-Butoxycarbonyl-L-Methionin-N-succinimidylester und 7,3 mmol 1.4 - Dihydro - 2.6 - dimethyl - 4 - (2 - trifluormethylphenyl) - pyridin - 3,5 - dicarbonsäure - 3 - (2 - aminoethyl) - ester - 5 - ethylester werden in 100 ml Methylenchlorid gelöst und eine halbe Stunde bei Raumtemperatur gerührt. Danach gibt man 7,3 mmol Triethylamin zu und rührt eine weitere Stunde bei Raumtemperatur. Danach wird die organische Phase nacheinander mit je 100 ml 10%ige Citronensäurelösung, Wasser, 10%ige Natriumhydrogencarbonatlösung, Wasser und mit Natriumchloridlösung ausgeschüttelt. Die organische Phase wird über Magnesiumsulfat getrocknet und eingeengt.

Ausbeute: 4,6 g.

Rf: 0.65 ($CHCl_3$/MeOH = 10:1).

Die nachfolgend aufgeführten Beispiele wurden analog Beispiel 1 hergestellt. Die Beispiele 8 und 9 wurden ausgehend von den jeweiligen enantiomerenreinen Dihydropyridinamin-Vorstufen synthetisiert.

Beispiel 2

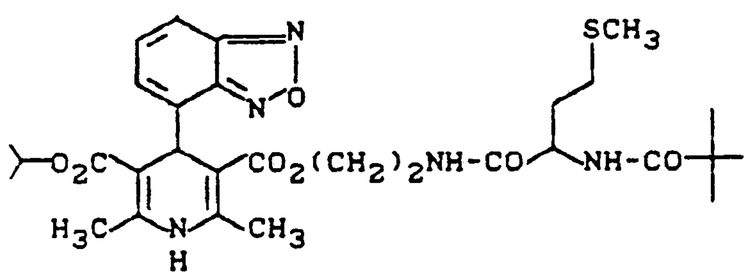

Ausbeute: 87,7%

$R_f$ : 0,408 ($CHCl_3$ / MeOH 10:1)

| Analyse : | C | H | N | S |
|---|---|---|---|---|
| ber. : | 57,0 | 6,5 | 11,1 | 5,1 |
| gef. : | 56,9 | 6,6 | 11,0 | 4,8 |

Beispiel 3

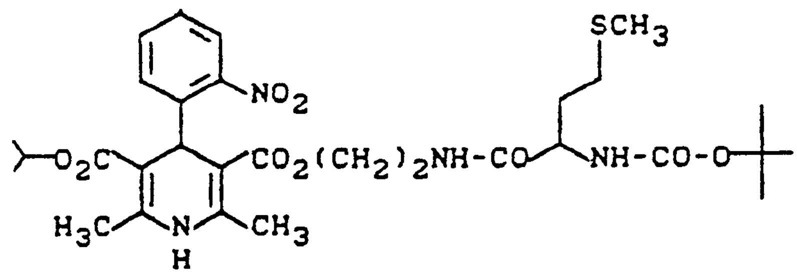

Ausbeute: 89,16%

$R_f$ : 0,362 ($CHCl_3$ / MeOH 10:1)

| Analyse : | C | H | N | S |
|---|---|---|---|---|
| ber. : | 56,8 | 6,7 | 8,8 | 5,1 |
| gef. : | 56,6 | 6,7 | 8,8 | 5,0 |

Beispiel 4

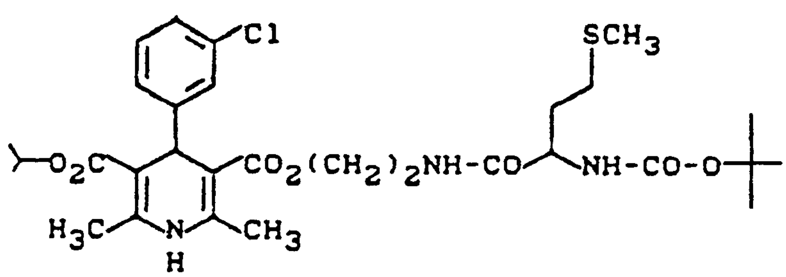

Ausbeute: 89%

$R_f$ : 0,42 ($CHCl_3$ / MeOH 10:1)

| Analyse : | C | H | N | S |
|---|---|---|---|---|
| ber. : | 57,7 | 6,8 | 6,7 | 5.1 |
| gef.: | 57,8 | 6,8 | 6,7 | 5,1 |

Beispiel 5

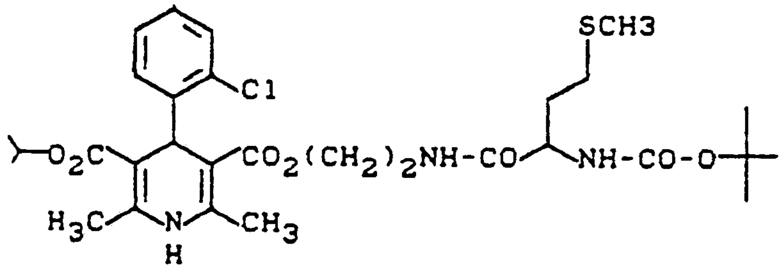

Ausbeute: 88,89%

$R_f$ : 0,484 ($CHCl_3$ / MeOH 10:1)

| Analyse : | C | H | N | S |
|---|---|---|---|---|
| ber. : | 57,7 | 6,8 | 6,7 | 5,1 |
| gef. : | 57,3 | 6,8 | 6,7 | 5,1 |

Beispiel 6

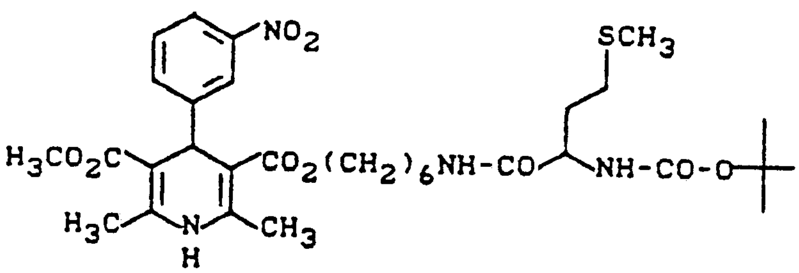

Ausbeute: 87,6%

$R_f$ : 0,475 ($CHCl_3$ / MeOH 10:1)

| Analyse : | C | H | N | S |
|---|---|---|---|---|
| ber. : | 58,0 | 7,0 | 8,5 | 4,8 |
| gef. : | 57,6 | 7,0 | 8,4 | 4,9 |

Beispiel 7

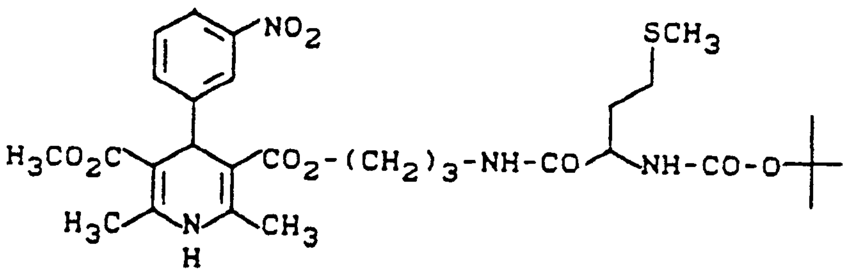

Ausbeute: 80,16%

$R_f$ : 0,429 ($CHCl_3$ / MeOH 10:1)

| Analyse : | C | H | N | S |
|---|---|---|---|---|
| ber. : | 56,1 | 6,9 | 9,0 | 5,2 |
| gef. : | 55,9 | 6,6 | 8,9 | 5,1 |

Beispiel 8

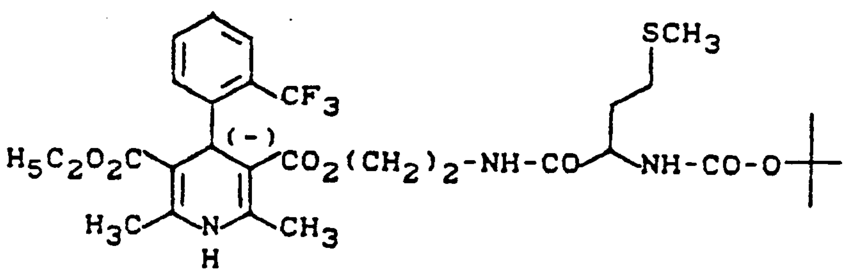

Ausbeute: 96,5%

$R_f$ : 0,65 ($CHCl_3$ / MeOH 10:1)

$[\alpha]^{20}_D$ : -86.06 in Ethanol

| Analyse : | C | H | F | N | S |
|---|---|---|---|---|---|
| ber. : | 55,5 | 6,1 | 8,86 | 6,5 | 4,98 |
| gef. : | 55,5 | 6,1 | 8,7 | 6,3 | 4,7 |

Beispiel 9

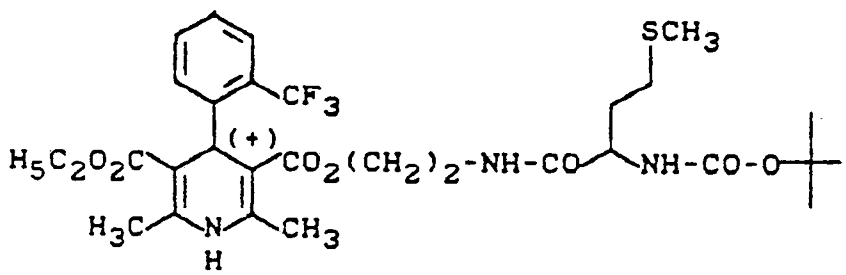

Ausbeute: 95,3%

$R_f$ : 0,64 ($CHCl_3$ / MeOH 10:1)

$[\alpha]^{20}_D$ : +53.7 in Ethanol

| Analyse: | C | H | F | N | S |
|---|---|---|---|---|---|
| ber.: | 55,98 | 6,26 | 8,86 | 6,5 | 4,98 |
| gef.: | 55,7 | 6,1 | 8,8 | 6,3 | 4,6 |

Beispiel 10

1,4-Dihydro-2,6-dimethyl-4-(2-trifluormethylphenyl)pyridin-3,5-dicarbonsäure-3-[2-(L-Methionyl)-aminoethyl]-ester-5-ethylester

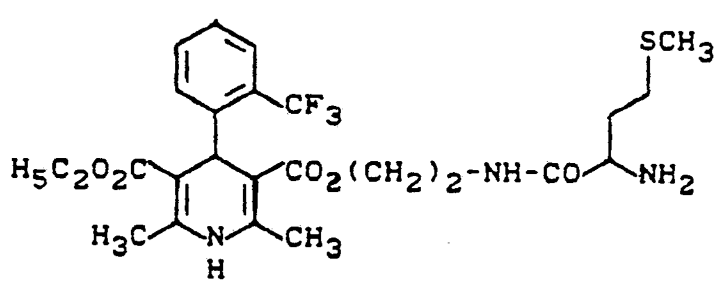

Zu 7,2 g (11,2 mmol) 1,4 - Dihydro - 2,6 - dimethyl - 4 - (2 - trifluormethylphenyl) - pyridin - 3,5 - dicarbonsäure - 3 - [2 - (N - tert - butyloxy - carbonyl - L - methionyl)aminoethyl] - ester - 5 - ethylester wurde innerhalb von 10 Minuten ein Gemisch aus 20 ml Trifluoressigsäure und 15 ml Methylenchlorid getropft.

Nach 2 Stunden Rühren bei Raumptemperatur wurde nacheinander mit 2 N NaOH und Wasser extrahiert und die organische Phase mit Natriumsulfat getrocknet und eingeengt.

Säulenchromatographie an Kieselgel (40—60 µm) mit Chloroform/MeOH/Ammoniak = 20/1/0,05 als

Eluierungsmittel lieferte nach Einengen das gewünschte Produkt als Schaum.
Ausbeute:( 4,1 g (67,3%).

| Analyse: | C | H | F | N | S |
|---|---|---|---|---|---|
| ber. : | 55,2 | 5,93 | 10,5 | 7,73 | 5,9 |
| gef. : | 55,1 | 5,8 | 10,3 | 7,5 | 5,8 |

**Patentansprüche**

1. Methioninsubstituierte Dihydropyridine der allgemeinen Formel (I)

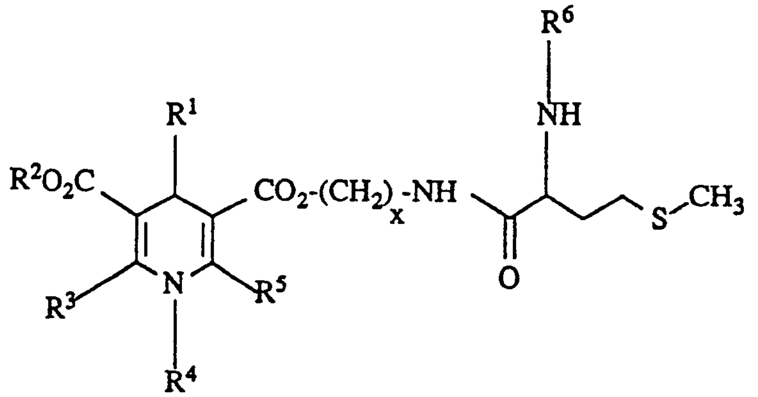

(I)

in welcher

$R^1$ fuř Phenyl, Thienyl, Furyl, Pyridyl, Chinolyl oder Benzoxadiazolyl steht, wobei die genannten Ringsysteme gegebenenfalls durch 1 bis 2 gleiche oder verschiedene Substituenten aus der Gruppe Phenyl, Alkyl mit 1 bis 2 Kohlenstoffatomen, Alkoxy mit 1 bis 2 Kohlenstoffatomen, Dioxymethylene, Fluor, Chlor, Brom oder Jod, Trifluormethyl, Trifluormethoxy, Difluormethoxy, Nitro, Cyano oder Azido, Trifluormethylsulfonyl oder Alkylmercapto mit 1 bis 4 Kohlenstoffatomen im Alkylrest substitiert sind,

$R^2$ einen geradkettigen, verzweigten oder cyclischen, gesättigten oder ungesättigten Kohlenwasserstoffrest mit bis zu 14 Kohlenstoffatomen darstellt, der gegebenenfalls durch ein Sauerstoffatom in der Kette unterbrochen ist und/oder der gegebenenfalls substituiert ist durch Fluor, Chlor, Brom, Jod, Cyano, Hydroxy, Acetoxy, Nitrooxy oder durch eine gegebenenfalls durch Fluor oder Chlor, Alkoxy mit 1 bis 4 Kohlenstoffatomen, Alkyl mit 1 bis 4 Kohlenstoffatomen, oder Trifluormethyl substituierte Phenyl- oder Phenoxygruppe oder durch eine α-, β- oder γ-Pyridylgruppe oder durch eine Aminogruppe, wobei diese Aminogruppe zwei gleiche oder verschiedene Substituenten aus der Gruppe Alkyl mit bis zu 4 Kohlenstoffatomen, Phenyl oder Benzyl trägt,

$R^3$ und $R^5$ gleich oder verschieden sind und jeweils für Wasserstoff, einen geradkettigen, verzweigten oder cyclischen Alkylrest mit bus zu 6 Kohlenstoffatomen, einen Phenyl- oder Benzylrest stehen, oder einen der Substituenten $R^3$ oder $R^5$ einen Alkylrest mit bis zu 4 Kohlenstoffatomen darstellt, der durch Acetoxy, Methoxy, oder Dimethoxy, Hydroxy, Amino, Phthalimido, Aminoalkoxy oder Phthalimidoalkoxy mit jeweils bis zu 4 Kohlenstoffatomen je Alkoxygruppe substituiert ist oder für die Formyl- oder Nitrilgruppe steht,

$R^4$ Wasserstoff oder einen Alkylrest mit bis zu 4 Kohlenstoffatomen bedeutet, der gegebenenfalls durch ein Sauerstoffatom in der Kette unterbrochen ist und/oder der gegebenenfalls durch einen Morpholino-Rest substituiert ist, oder einen Phenyl- oder Benzylrest darstellt,

$R^6$ für Wasserstoff oder eine Aminoschutzgruppe aus der Gruppe Vinyl, Allyl, tert.-Butyloxycarbonyl, Benzyl, Benzyloxycarbonyl, 2-Nitro- bzw. 4-Nitrobenzyl, 2-Nitro- bzw. 4-Nitro-benzyloxycarbonyl, 4-Methoxyphenyl, Formyl, Benzoyl, Acetyl, Chloracetyl, Trichloracetyl, Trifluoracetyl, Methyloxycarbonyl, Allyloxycarbonyl, Trimethyl-, Triethyl-bzw. Triphenylsilyl, tert.-Butyl-dimethylsilyl, 2,4-Dimethoxybenzyl, 2,4-Dimethoxybenzyloxycarbonyl, 4-Methoxybenzyl, 4-Methoxymethyloxyphenyl, Bis(4-Methoxyphenyl)-methyl, tert.-Butoxycarbonylmethyl, Allyloxycarbonylmethyl, Methoxymethyl, Methylthiomethyl, Methoxyethoxymethyl, [2-(Trimethylsilyl)ethoxy]-methyl, 2-(Methylthiomethoxy)ethoxycarbonyl oder Tetrahydropyranyl steht, und

X für eine Zahl von 2 bis 12 steht,

und deren physiologisch unbedenklichen Salze.

2. Verbindungen der allgemeinen Formel (I) gemäß Anspruch 1, in welcher

$R^1$ für Phenyl, Pyridyl oder Benzoxadiazolyl steht, wobei der Phenylring durch 1 oder 2 gleiche oder verschiedene Substituenten aus der Gruppe Chlor, Trifluormethyl, Nitro oder Cyano substituiert ist,

$R^2$ einen geradkettigen, verzweigten oder cyclischen Kohlenwasserstoffrest mit bis zu 7 Kohlenstoffatomen darstellt, der gegebenenfalls durch ein Sauerstoffatom in der Kette unterbrochen ist und/oder der gegebenenfalls substituiert ist durch Fluor, Cyano, Acetoxy, Phenyl, Phenoxy, α-, β- oder γ-Pyridyl oder durch eine Aminogruppe, wobei diese Aminogruppe zwei gleiche oder verschiedene Substituenten aus der Gruppe Alkyl mit bis zu 4 Kohlenstoffatomen und Benzyl trägt,

$R^3$ und $R^5$ gleich oder verschieden sind und jeweils für einen geradkettigen oder cyclischen Alkylrest mit bis zu 6 Kohlenstoffatomen stehen, oder

einer der Substituenten $R^3$ oder $R^5$ einen Alkylrest mit bis zu 2 Kohlenstoffatomen darstellt, der durch Acetoxy, Hydroxy, Phthalimido, Amino, Phthalimidoethoxy oder Aminoethoxy substituiert ist,

oder für die Formyl- oder Nitrilgruppe steht,

$R^4$ für Wasserstoff oder den Morpholinoethylrest steht,

X für eine Zahl von 2 bis 8 steht,

und deren physiologisch unbedenklichen Salze.

3. Verfahren zur Herstelung von Verbindungen der allgemeinen Formel (I)

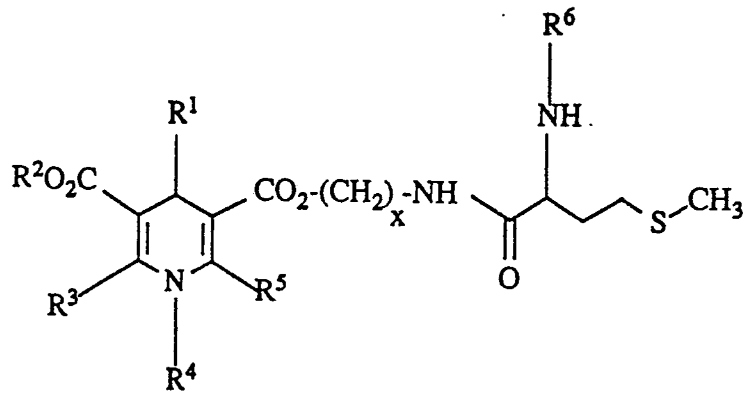

(I)

$R^1$ für Phenyl, Thienyl, Furyl, Pyridyl, Chinolyl oder Benzoxadiazolyl steht, wobei die genannten Ringsysteme gegebenenfalls durch 1 bis 2 gleiche oder verschiedene Substituenten aus der Gruppe Phenyl, Alkyl mit 1 bis 2 Kohlenstoffatomen, Alkoxy mit 1 bis 2 Kohlenstoffatomen, Dioxymethylene, Fluor, Chlor, Brom oder Jod, Trifluormethyl, Trifluormethoxy, Difluormethoxy, Nitro, Cyano oder Azido, Trifluormethylsulfonyl oder Alkylmercapto mit 1 bis 4 Kohlenstoffatomen im Alkylrest substituiert sind,

$R^2$ einen geradkettigen, verzweigten oder cyclischen, gesättigten oder ungesättigten Kohlenwasserstoffrest mit bis zu 14 Kohlenstoffatomen darstellt, der gegebenenfalls durch ein Sauerstoffatom in der Kette unterbrochen ist und/oder der gegebenenfalls substituiert ist durch Fluor, Chlor, Brom, Jod, Cyano, Hydroxy, Acetoxy, Nitrooxy oder durch eine gegebenenfalls durch Fluor oder Chlor, Alkoxy mit 1 bis 4 Kohlenstoffatomen, Alkyl mit 1 bis 4 Kohlenstoffatomen, oder Trifluormethyl substituierte Phenyl- oder Phenoxygruppe oder durch eine α-, β- oder γ-Pyridylgruppe oder durch eine Aminogruppe, wobei diese Aminogruppe zwei gleiche oder verschiedene Substituenten aus der Gruppe Alkyl mit bis zu 4 Kohlenstoffatomen, Phenyl oder Benzyl trägt,

$R^3$ und $R^5$ gleich oder verschieden sind und jeweils für Wasserstoff, einen geradkettigen, verzweigten oder cyclischen Alkylrest mit bus zu 6 Kohlenstoffatomen, einen Phenyl- oder Benzylrest stehen, oder einen der Substituenten $R^3$ oder $R^5$ einen Alkylrest mit bis zu 4 Kohlenstoffatomen darstellt, der durch Acetoxy, Methoxy, oder Dimethoxy, Hydroxy, Amino, Phthalimido, Aminoalkoxy oder Phthalimidoalkoxy mit jeweils bis zu 4 Kohlenstoffatomen je Alkoxygruppe substituiert ist oder für die Formyl- oder Nitrilgruppe steht,

$R^4$ Wasserstoff oder einen Alkylrest mit bis zu 4 Kohlenstoffatomen bedeutet, der gegebenenfalls durch ein Sauerstoffatom in der Kette unterbrochen ist und/oder der gegebenenfalls durch einen Morpholino-Rest substituiert ist, oder einen Phenyl- oder Benzylrest darstellt,

$R^6$ für Wasserstoff oder eine Aminoschutzgruppe aus der Gruppe Vinyl, Allyl, tert.-Butyloxycarbonyl, Benzyl, Benzyloxycarbonyl, 2-Nitro- bzw. 4-Nitrobenzyl, 2-Nitro- bzw. 4-Nitro-benzyloxycarbonyl, 4-Methoxyphenyl, Formyl, Benzoyl, Acetyl, Chloracetyl, Trichloracetyl, Trifluoracetyl, Methyloxycarbonyl, Allyloxycarbonyl, Trimethyl-, Triethyl-bzw. Triphenylsilyl, tert.-Butyl-dimethylsilyl, 2,4-Dimethoxybenzyl, 2,4-Dimethoxybenzyloxycarbonyl, 4-Methoxybenzyl, 4-Methoxymethyloxyphenyl, Bis(4-Methoxyphenyl)-methyl, tert.-Butoxycarbonylmethyl, Allyloxycarbonylmethyl, Methoxymethyl, Methylthiomethyl, Methoxyethoxymethyl, [2-(Trimethylsilyl)ethoxy]-methyl, 2-(Methylthiomethoxy)ethoxycarbonyl oder Tetrahydropyranyl steht, und

X für eine Zahl von 2 bis 12 steht,
dadurch gekennzeichnet, daß man Methioninverbindungen der allgemeinen Formel (II)

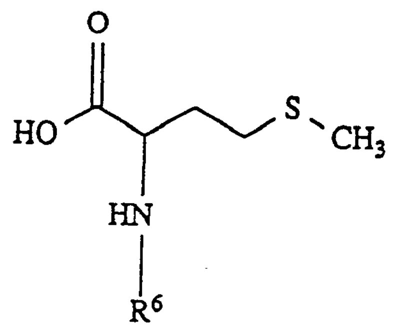

(II)

in welcher
$R^6$ für eine Aminoschutzgruppe mit der oben angegebenen Bedeutung steht, nach Aktivierung der Carboxylgruppe mit Dihydropyridinen der allgemeinen Formel (III),

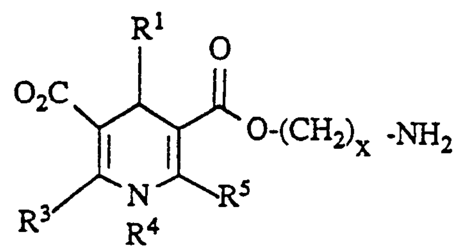

(III)

in welcher
$R^1$ bis $R^5$ und X die oben angegebene Bedeutung haben,
in einem inerten Lösungsmittel bei Temperaturen zwischen −30°C und +60°C umsetzt, gegebenenfalls vorhandene Schutzgruppen abspaltet und anschließend gegebenenfalls durch Umsetzen mit Säuren physiologisch unbedenkliche Salze herstellt.

4. Verfahren gemäß Anspruch 3, dadurch gekennzeichnet, daß man die Reaktion bei −20°C bis +20°C durchführt.

5. Verfahren gemäß Anspruch 3, dadurch gekennzeichnet, daß man die Umsetzung bei einem pH von 7 bis 11 durchführt.

6. Verfahren gemäß Anspruch 3, dadurch gekennzeichnet, daß man die Crboxylgruppe in Verbindungen der Formel (II) aktiviert, in dem man Anhydride mit Pivaloylchlorid oder Chlorameisensäureestern herstellt oder das Mesylat mittels Methansulfonsäurechlorid herstellt oder die Aktivierung mit 1-Hydroxybenztriazol, N-Hydroxysuccinimid oder Dicyclohexylcarbodiimid durchführt und anschließend die aktivierte Carboxylgruppe mit Dihydropyridinen der Formel (III) umsetzt.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls mit üblichen Hilfs- und Trägerstoffen in eine geeignete Applikationsform überführt.

9. Verwendung von Verbindungen der allgemeinen Formel (I) gemäß Anspruch 1 zur Herstellung von Kreislaufmitteln.

**Revendications**

1. Dihydropyridines à substituant méthionine, répondant à la formule générale (I)

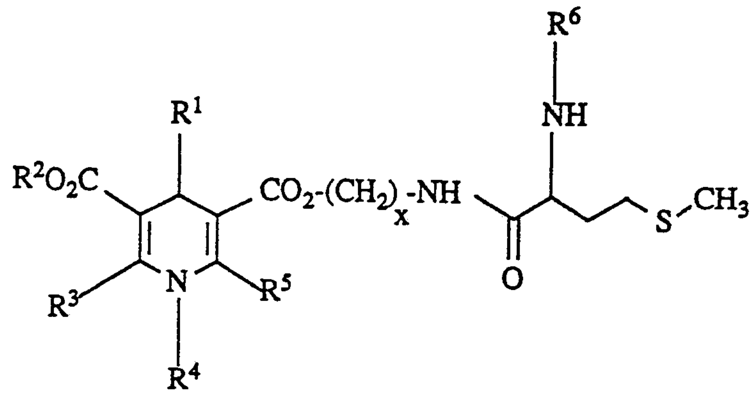

(I)

dans laquelle

$R^1$ représente un groupe phényle, thiényle, furyle, pyridyle, quinolyle ou benzoxadiazolyle, les systèmes cycliques cités pouvant éventuellement être substitués par 1 ou 2 substituants, identiques ou différents, choisis parmi un reste phényle, alkyle ayant 1 à 2 atomes de carbone, alcoxy ayant 1 à 2 atomes de carbone, dioxyméthylène, par un atome de fluor, de chlore, de brome ou d'iode, par un groupe trifluorométhyle, trifluorométhoxy, difluorométhoxy, nitro, cyano ou azido, trifluorométhylsulfonyle ou alkylmercapto (comportant 1 à 4 atomes de carbone dans le reste alkyle),

$R^2$ représente un reste d'hydrocarbure l'inéaire, ramifié ou cyclique, saturé ou insaturé, comportant jusqu'à 14 atomes de carbone (dont la chaîne peut éventuellement être interrompue par un atome d'oxygène et/ou qui peut éventuellement être substituée par du fluor, du chlore, du brome, de l'iode, par un groupe cyano, hydroxyle, acétoxy, nitro-oxy ou par un groupe phényle ou phénoxy (lui-même éventuellement substitué par du fluor ou par du chlore, par un groupe alcoxy ayant 1 à 4 atomes de carbone, alkyle ayant 1 à 4 atomes de carbone ou trifluorométhyle) ou par un groupe α-, β- ou γ-pyridyle ou par un groupe amino, ce groupe amino portant 2 substituants, identiques ou différents, choisis parmi un groupe alkyle ayant jusqu'à 4 atomes de carbone, un groupe phényle ou benzyle,

$R^3$ et $R^5$ sont identiques ou différents et représentent chacun un atome d'hydrogène, un reste alkyle ayant jusqu'à 6 atomes de carbone et qui est linéaire, ramifié ou cyclique, un reste phényle ou benzyle, ou bien l'un des substituants $R^3$ ou $R^5$ représente un reste alkyle ayant jusqu'à 4 atomes de carbone (qui est substitué par un groupe acétoxy, méthoxy ou diméthoxy, hydroxy, amino, phtalimido, aminoalcoxy ou phtalimidoalcoxy ayant chacun jusqu'à 4 atomes de carbone par groupe alcoxy) ou bien ce symbole représente un groupe formule ou nitrile,

$R^4$ représente un atome d'hydrogène ou un reste alkyle ayant jusqu'à 4 atomes de carbone (dont la chaîne est éventuellement interrompue par un atome d'oxygène et/ou qui est éventuellement substituée par un reste morpholino), ou bien $R^4$ représente un reste phényle ou benzyle,

$R^6$ représente un atome d'hydrogène ou un groupe protecteur de fonction amino, choisi parmi un groupe vinyle, allyle, tertiobutyloxycarbonyle, benzyle, benzyloxycarbonyle, 2-nitro- ou 4-nitro-benzyle, 2-nitro- ou 4-nitro-benzyloxycarbonyle, 4-méthoxyphényle, formyle, benzoyle, acétyle, chloracétyle, trichloracétyle, trifluoracétyle, méthyloxycarbonyle, allyloxycarbonyle, triméthyl-, triéthyl- ou triphénylsilyle, tertiobutyl-diméthylsilyle, 2,4-diméthoxybenzyle, 2,4-diméthoxybenzyloxycarbonyle, 4-méthoxybenzyle, 4-méthoxyméthyloxyphényle, bis-(4-méthoxyphényl)méthyle, tertiobutoxycarbonyl-méthyle, allyloxycarbonylméthyle, méthoxyméthyle, méthylthiométhyle, méthoxyéthoxyméthyle, [2-(triméthylsilyl)éthoxy]-méthyle, 2-(méthylthiométhoxy)-éthoxycarbonyle ou tétrahydropyrannyle, et

x représente un nombre valant 2 à 12,

et leurs sels ne présentant pas d'inconvénients psysiologiques.

2. Composés de formule générale (I) selon la revendication 1, dans laquelle

$R^1$ représente un groupe phényle, pyridyle ou benzoxadiazolyle, le noyau phényle étant substitué par 1 ou 2 substituants, identiques ou différents, choisis parmi un atome de chlore, un groupe trifluorométhyle, nitro ou cyano,

$R^2$ représente un reste d'hydrocarbure linéaire, ramifié ou cyclique ayant jusqu'à 7 atomes de carbone, dont la chaîne peut éventuellement être interrompue par un atome d'oxygène et/ou qui est éventuellement substituée par du fluor, par un groupe cyano, acétoxy, phényle, phénoxy, α-, β- ou γ-pyridyle ou par un groupe amino, ce groupe amino portant deux substituants, identiques ou différents, choisis parmi un groupe alkyle ayant jusqu'à 4 atomes de carbone et un groupe benzyle,

$R^3$ et $R^5$ sont identiques ou différents et représentent chacun ou reste alkyle linéaire ou cyclique ayant jusqu'à 6 atomes de carbone, ou bien

l'un des substituants $R^3$ ou $R^5$ représente un reste alkyle ayant jusqu'à 2 atomes de carbone, qui est substitué par un groupe acétoxy-hydroxy, phtalimido, amino, phtalimido-éthoxy ou aminoéthoxy, ou bien il représente le groupe formyle ou nitrile,

$R^4$ représente de préférence un atome d'hydrogène ou le reste morpholino-éthyle,

$R^6$ représente un atome d'hydrogène ou un groupe protecteur de fonction amino, choisi parmi un groupe tertio-butyloxycarbonyle, benzyloxycarbonyle, phtalimido ou triméthylsilyle, et

x représente un nombre valant 2 à 8,

et leurs sels dépourvus d'inconvénients physiologiques.

3. Procédé pour préparer des composés de formule générale (I)

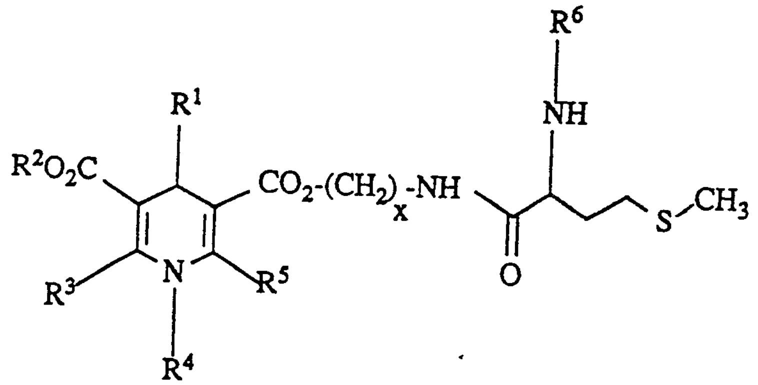

(I)

dans laquelle

$R^1$ représente un groupe phényle, thiényle, furyle, pyridyle, quinolyle ou benzoxadiazolyle, les systèmes cycliques cités pouvant éventuellement être substitués par 1 ou 2 substituants, identiques ou différents, choisis parmi un reste phényle, alkyle ayant 1 à 2 atomes de carbone, alcoxy ayant 1 à 2 atomes de carbone, dioxyméthylène, par un atome de fluor, de chlore, de brome ou d'iode, par un groupe trifluorométhyle, trifluorométhoxy, difluorométhoxy, nitro, cyano ou azido, trifluorométhylsulfonyle ou alkylmercapto (comportant 1 à 4 atomes de carbone dans le reste alkyle),

$R^2$ représente un reste d'hydrocarbure l'inéaire, ramifié ou cyclique, saturé ou insaturé, comportant jusqu'à 14 atomes de carbone (dont la chaîne peut éventuellement être interrompue par un atome d'oxygène et/ou qui peut éventuellement être substituée par du fluor, du chlore, du brome, de l'iode, par un groupe cyano, hydroxyle, acétoxy, nitro-oxy ou par un groupe phényle ou phénoxy (lui-même éventuellement substitué par du fluor ou par du chlore, par un groupe alcoxy ayant 1 à 4 atomes de carbone, alkyle ayant 1 à 4 atomes de carbone ou trifluorométhyle) ou par un groupe α-, β- ou γ-pyridyle ou par un groupe amino, ce groupe amino portant 2 substituants, identiques ou différents, choisis parmi un groupe alkyle ayant jusqu'à 4 atomes de carbone, un groupe phényle ou benzyle,

$R^3$ et $R^5$ sont identiques ou différents et représentent chacun un atome d'hydrogène, un reste alkyle ayant jusqu'à 6 atomes de carbone et qui est linéaire, ramifié ou cyclique, un reste phényle ou benzyle, ou bien l'un des substituants $R^3$ ou $R^5$ représente un reste alkyle ayant jusqu'à 4 atomes de carbone (qui est substitué par un groupe acétoxy, méthoxy ou diméthoxy, hydroxy, amino, phtalimido, aminoalcoxy ou phtalimidoalcoxy ayant chacun jusqu'à 4 atomes de carbone par groupe alcoxy) ou bien ce symbole représente un groupe formule ou nitrile,

$R^4$ représente un atome d'hydrogène ou un reste alkyle ayant jusqu'à 4 atomes de carbone (dont la chaîne est éventuellement interrompue par un atome d'oxygène et/ou qui est éventuellement substituée par un reste morpholino), ou bien $R^4$ représente un reste phényle ou benzyle,

$R^6$ représente un atome d'hydrogène ou un groupe protecteur de fonction amino, choisi parmi un groupe vinyle, allyle, tertiobutyloxycarbonyle, benzyle, benzyloxycarbonyle, 2-nitro- ou 4-nitro-benzyle, 2-nitro- ou 4-nitro-benzyloxycarbonyle, 4-méthoxyphényle, formyle, benzoyle, acétyle, chloracétyle, trichloracétyle, trifluoracétyle, méthyloxycarbonyle, allyloxycarbonyle, triméthyl-, triéthyl- ou triphénylsilyle, tertiobutyl-diméthylsilyle, 2,4-diméthoxybenzyle, 2,4-diméthoxybenzyloxycarbonyle, 4-méthoxybenzyle, 4-méthoxyméthyloxyphényle, bis-(4-méthoxyphényl)méthyle, tertiobutoxycarbonyl-méthyle, allyloxycarbonylméthyle, méthoxyméthyle, méthylthiométhyle, méthoxyéthoxyméthyle, [2-(triméthylsilyl)éthoxy]-méthyle, 2-(méthylthiométhoxy)-éthoxycarbonyle ou tétrahydropyrannyle, et

x représente un nombre valant 2 à 12,
procédé caractérisé en ce qu'on fait réagir des méthionines de formule générale (II)

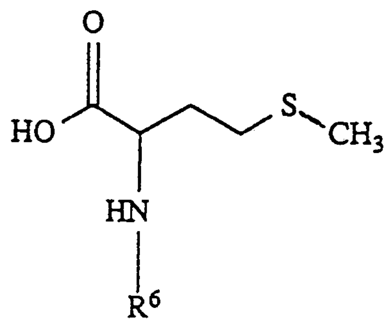

(II)

dans laquelle
$R^6$ représente un groupe protecteur de la fonction amino, ayant le sens indiqué ci-dessus, après activation du groupe carboxyle, avec des dihydropyridines de formule générale (III)

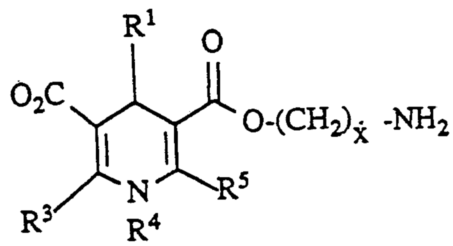

(III)

dans laquelle
$R^1$ à $R^5$ et x ont les sens indiqués ci-dessus, en opérant dans un solvant inerte à des températures comprises entre −30°C et +60°C, on scinde des groupes protecteurs éventuellement présents, puis, éventuellement par réaction avec des acides, on prépare des sels sans inconvénients physiologiques.

4. Procédé selon la revendication 3, caractérisé en ce qu'on conduit la réaction entre −20°C et +20°C.

5. Procédé selon la revendication 3, caractérisé en ce qu'on conduit la réaction à un pH de 7 à 11.

6. Procédé selon la revendication 3, caractérisé en ce qu'on active le groupe carboxyle de composés de formule (II), en préparant des anhydrides avec le chlorure de pivaloyle ou des esters de l'acide chloroformique et/ou l'on prépare le mésylate à l'aide du chlorure de l'acide méthane sulfonique ou l'on conduit l'activation avec du 1-hydroxybenzotriazole, du N-hydroxysuccinimide ou du dicyclohexylcarbodiimide, puis on fait réagir le groupe carboxyle, ainsi activé, avec des dihydropyridines de formule (III).

7. Médicament contenant au monis un composé de formule générale (I) selon la revendication 1.

8. Procédé pour préparer des médicaments, caractérisé en ce qu'on met des composés de formule générale (I) selon la revendication 1, éventuellement à l'aide adjuvants et excipients, supports ou véhicules usuels, sous une forme convenant bien pour l'administration.

9. Utilisation de composés de formulé générale (I) selon la revendication 1 pour préparer des produits agissant sur la circulation du sang.

**Claims**

1. Methionine-substituted dihydropyridines of the general formula (I)

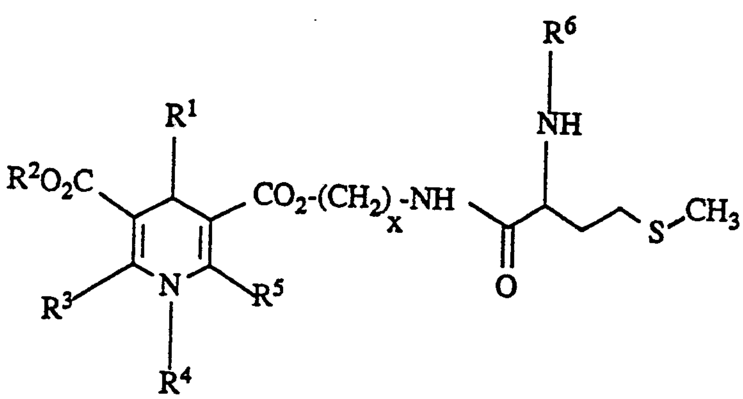

(I)

in which

$R^1$ represents phenyl, thienyl, furyl, pyridyl, quinolyl or benzoxadiazolyl, the said ring systems optionally being substituted by 1 to 2 identical or different substituents from the group comprising phenyl, alkyl having 1 to 2 carbon atoms, alkoxy having 1 to 2 carbon atoms, dioxymethylene, fluorine, chlorine, bromine or iodine, trifluoromethyl, trifluoromethoxy, difluoromethoxy, nitro, cyano or azido, trifluoromethylsulphonyl or alkylmercapto having 1 to 4 carbon atoms in the alkyl radical,

$R^2$ represents a straight-chain, branched or cyclic, saturated or unsaturated, hydrocarbon radical which has up to 14 carbon atoms, which is optionally interrupted in the chain by one oxygen atom and/or which is optionally substituted by fluorine, chlorine, bromine, iodine, cyano, hydroxyl, acetoxy, nitrooxy or by a phenyl or phenoxy group which is optionally substituted by fluorine or chlorine, alkoxy having 1 to 4 carbon atoms, alkyl having 1 to 4 carbon atoms or trifluoromethyl, or $R^2$ is substituted by an α-, β- or γ-pyridyl group or by an amino group, this amino group carrying two identical or different substituents from the group comprising alkyl having up to 4 carbon atoms, phenyl or benzyl,

$R^3$ and $R^5$ are identical or different and each represents hydrogen, a straight-chain, branched or cyclic alkyl radical having up to 6 carbon atoms, a phenyl or benzyl radical, or one of the substituents $R^3$ or $R^5$ represents an alkyl radical which has up to 4 carbon atoms and which is substituted by acetoxy, methoxy, or dimethoxy, hydroxyl, amino, phthalimido, aminoalkoxy or phthalimidoalkoxy having, in each case, up to 4 carbon atoms in each alkoxy group, or represents the formyl or nitrile group,

$R^4$ denotes hydrogen or an alkyl radical which has up to 4 carbon atoms, which is optionally interrupted in the chain by one oxygen atom and/or which is optionally substituted by a morpholino radical, or represents a phenyl or benzyl radical, $R^6$ represents hydrogen or an amino protective group from the group comprising vinyl, allyl, tert.-butyloxycarbonyl, benzyl, benzyloxycarbonyl, 2-nitro- or 4-nitro-benzyl, 2-nitro- or 4-nitro-benzyloxycarbonyl, 4-methoxyphenyl, formyl, benzoyl, acetyl, chloroacetyl, trichloroacetyl, trifluoroacetyl, methyloxycarbonyl, allyloxycarbonyl, trimethyl-, triethyl- or triphenylsilyl, tert.-butyl-dimethylsilyl, 2,4-dimethoxybenzyl, 2,4-dimethoxybenzyloxycarbonyl, 4-methoxybenzyl, 4-methoxymethyloxyphenyl, bis(4-methoxyphenyl)methyl, tert.-butoxycarbonylmethyl, allyloxycarbonylmethyl, methoxymethyl,methylthiomethyl, methoxyethoxymethyl, [2-(trimethylsilyl)ethoxy]-methyl, 2-(methylthiomethoxy)ethoxycarbonyl or tetra.-hydropyranyl, and

X represents a number from 2 to 12, and their physiologically acceptable salts.

2. Compounds of the general formula (I) according to Claim 1, in which

$R^1$ represents phenyl, pyridyl or benzoxadiazolyl, the phenyl ring being substituted by 1 or 2 identical or different substituents from the group comprising chlorine, trifluoromethyl, nitro or cyano,

$R^2$ represents a straight-chain, branched or cyclic hydrocarbon radical which has up to 7 carbon atoms, which is optionally interrupted in the chain by one oxygen atom and/or which is optionally substituted by fluorine, cyano, acetoxy, phenyl, phenoxy, α-, β- or γ-pyridyl or by an amino group, this amino group carrying two identical or different substituents from the group comprising alkyl having up to 4 carbon atoms and benzyl, $R^3$ and $R^5$ are identical or different and each represents a straight-chain or cyclic alkyl radical having up to 6 carbon atoms, or one of the substituents $R^3$ or $R^5$ represents an alkyl radical which has up to 2 carbon atoms and which is substituted by acetoxy, hydroxyl, phthalimido, amino, pthalimidoethoxy or aminoethoxy, or represents the formyl or nitrile group,

$R^4$ preferably represents hydrogen or the morpholinoethyl radical,

X represents a number from 2 to 8, and their physiologially acceptable salts.

3. Process for the preparation of compounds of the general formula (I)

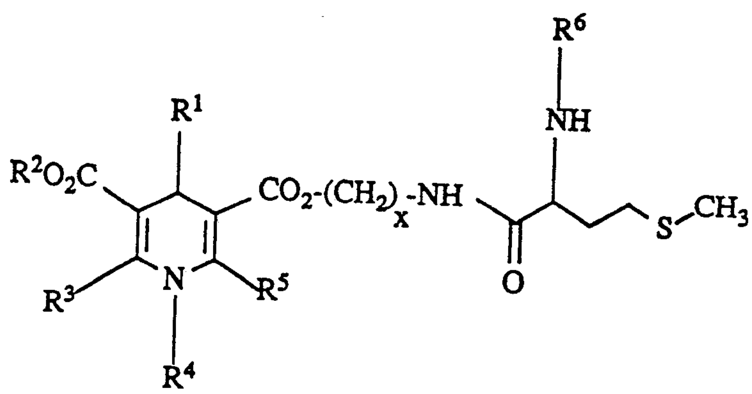

(I)

in which

$R^1$ represents phenyl, thienyl, furyl, pyridyl, quinolyl or benzoxadiazolyl, the said ring systems optionally being substituted by 1 to 2 identical or different substituents from the group comprising phenyl, alkyl having 1 to 2 carbon atoms, alkoxy having 1 to 2 carbon atoms, dioxymethylene, fluorine, chlorine, bromine or iodine, trifluoromethyl, trifluoromethoxy, difluoromethoxy, nitro, cyano or azido,

trifluoromethylsulphonyl or alkylmercapto having 1 to 4 carbon atoms in the alkyl radical,

$R^2$ represents a straight-chain, branched or cyclic, saturated or unsaturated, hydrocarbon radical which has up to 14 carbon atoms, which is optionally interrupted in the chain by one oxygen atom and/or which is optionally substituted by fluorine, chlorine, bromine, iodine, cyano, hydroxyl, acetoxy, nitrooxy or by a phenyl or phenoxy group which is optionally substituted by fluorine or chlorine, alkoxy having 1 to 4 carbon atoms, alkyl having 1 to 4 carbon atoms or trifluoromethyl, or $R^2$ is substituted by an α-, β- or γ-pyridyl group or by an amino group, this amino group carrying two identical or different substituents from the group comprising alkyl having up to 4 carbon atoms, phenyl or benzyl,

$R^3$ and $R^5$ are identical or different and each represents hydrogen, a straight-chain, branched or cyclic alkyl radical having up to 6 carbon atoms, a phenyl or benzyl radical, or one of the substituents $R^3$ or $R^5$ represents an alkyl radical which has up to 4 carbon atoms and which is substituted by acetoxy, methoxy, or dimethoxy, hydroxyl, amino, phthalimido, aminoalkoxy or phthalimidoalkoxy having, in each case, up to 4 carbon atoms in each alkoxy group, or represents the formyl or nitrile group,

$R^4$ denotes hydrogen or an alkyl radical which has up to 4 carbon atoms, which is optionally interrupted in the chain by one oxygen atom and/or which is optionally substituted by a morpholino radical, or represents a phenyl or benzyl radical,

$R^6$ represents hydrogen or an amino protective group from the group comprising vinyl, allyl, tert.-butyloxycarbonyl, benzyl, benzyloxycarbonyl, 2-nitro- or 4-nitro-benzyl, 2-nitro- or 4-nitro-benzyloxycarbonyl, 4-methoxyphenyl, formyl, benzoyl, acetyl, chloroacetyl, trichloroacetyl, trifluoroacetyl, methyloxycarbonyl, allyloxycarbonyl, trimethyl-, triethyl- or triphenylsilyl, tert.-butyl-dimethylsilyl, 2,4-dimethoxybenzyl, 2,4-dimethoxybenzyloxycarbonyl, 4-methoxybenzyl, 4-methoxymethyloxyphenyl, bis(4-methoxyphenyl)methyl, tert.-butoxycarbonylmethyl, allyloxycarbonylmethyl, methoxymethyl, methylthiomethyl, methoxyethoxymethyl, [2-(trimethylsilyl)ethoxy]-methyl, 2-(methylthiomethoxy)-ethoxycarbonyl or tetra.-hydropyranyl, and

X represents a number from 2 to 12, characterized in that methionine compounds of the general formula (II)

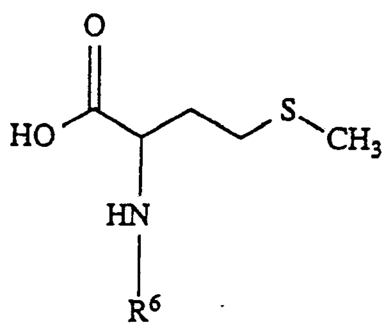

(II)

in which $R^6$ represents an amino protective group with the above meaning, are reacted, after activation of the carboxyl group, with dihydropyridines of the general formula (III)

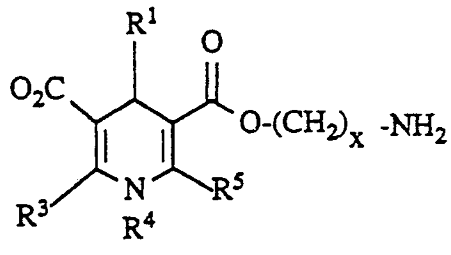

(III)

in which

$R^1$ to $R^5$ and X have the abovementioned meaning, in an inert solvent at temperatures between −30°C and +60°C, where appropriate protective groups which are present are eliminated, and then, where appropriate, physiologically acceptable salts are prepared by reaction with acids.

4. Process according to Claim 3, characterised in that the reaction is carried out at −20°C to +20°C.

5. Process according to Claim 3, characterised in that the reaction is carried out at a pH of 7 to 11.

6. Process according to Claim 3, characterised in that the carboxyl group in compounds of the formula (II) is activated by preparing anhydrides with pivaloyl chloride or chloroformic esters, or the mesylate is prepared by use of methanesulphonyl chloride, or the activation is carried out with 1-hydroxybenzotriazole, N-hydroxysuccinimide or dicyclohexylcarbodiimide, and then the activated carboxyl group is reacted with dihydropyridines of the formula (III).

7. Medicament containing at least one compound of the general formula (I) according to Claim 1.

8. Process for the preparation of medicaments, characterized in that compounds of the general formula (I) according to Claim 1 are converted into a suitable form for administration, where appropriate with customary auxiliaries and vehicles.

9. Use of compounds of the general formula (I) according to Claim 1 for the preparation of cardiovascular agents.